# FASCICULE DE BREVET EUROPEEN

(11) **EP 1 385 858 B1**
(45) Date de publication et mention de la délivrance du brevet: **12.04.2006**
(21) Numéro de dépôt: 02738222.5
(22) Date de dépôt: 07.05.2002
(51) Int. Cl.: C07H 17/075, A61K 31/7048, A61P 7/02

(54) **NOUVEAUX DERIVES 5-THIO-SS-D-XYLOPYRANOSIDES, PROCEDE DE PREPARATION, COMPOSITIONS PHARMACEUTIQUES LES CONTENANT ET LEUR UTILISATION EN THERAPEUTIQUE**
NEUE 5-THIO-SS-D-XYLOPYRANOSIDDERIVATE, VERFAHREN ZU DEREN HERSTELLUNG, DIESE ENTHALTENDE PHARMAZEUTISCHE ZUSAMMENSETZUNGEN UND DEREN THERAPEUTISCHE VERWENDUNG
NOVEL 5-THIO-SS-D-XYLOPYRANOSIDE DERIVATIVES, PREPARATION METHOD THEREOF, PHARMACEUTICAL COMPOSITIONS CONTAINING SAME AND THE THERAPEUTIC USE THEREOF

(30) Priorité: 11.05.2001 FR 0106236
(43) Date de publication de la demande: 04.02.2004
(73) Titulaire: LABORATOIRES FOURNIER S.A., 21300 Chenove (FR)
(72) Inventeur: BARBEROUSSE, Véronique, F-21121 Hauteville-Les-Dijon (FR); SAMRETH, Soth, F-21121 Daix (FR)
(74) Mandataire: Gillard, Marie-Louise
(86) Numéro de dépôt international: PCT/FR2002/001573
(87) Numéro de publication internationale: WO 2002/092614

(56) Documents cités:
- EP-A- 0 421 829

## Description

La présente invention concerne de nouveaux dérivés du 4-méthyl-2-oxo-2*H*-1-benzopyran-7-yl 5-thio-β-D-xylopyranoside, leur utilisation pour la préparation de médicaments antithrombotiques, ainsi que les compositions pharmaceutiques les contenant. L'invention a également pour objet un procédé de préparation de ces composés.

Le brevet EP 421 829 décrit des benzopyranone β-D-thioxylosides de formule (A) : 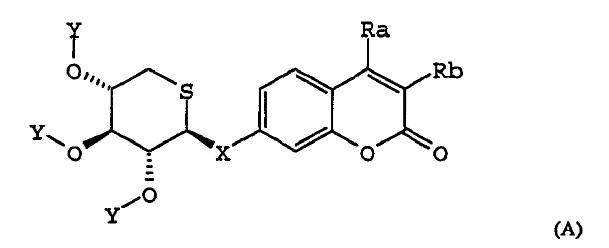 dans laquelle :
X est O ou S,
Y est notamment un atome d'hydrogène ou le groupe -COCH₃,
Ra est notamment un groupe (C₁-C₄) alkyle, et
Rb est en particulier un atome d'hydrogène, un groupe (C₁-C₄) alkyle ou un atome d'halogène.

Ces composés sont utiles dans le traitement et la prévention des maladies liées aux troubles circulatoires, notamment en tant qu'agents antithrombotiques veineux.

Toutefois, les composés décrits dans EP 421 829 présentent une solubilité insuffisante, notamment dans des solvants physiologiquement acceptables, pour permettre leur utilisation par voie injectable. De ce fait, leur utilisation n'est pas possible dans le cas d'administration d'urgence ou sur des patients inconscients, chez lesquels la voie injectable est la seule voie d'administration possible ou si, par commodité, il est préférable d'administrer l'un de ces composés en association avec d'autres médicaments par perfusion.

La présente invention concerne de nouveaux dérivés du 4-méthyl-2-oxo-2*H*-1-benzopyran-7-yl 5-thio-β-D-xylopyranoside ayant une activité antithrombotique. Ces composés sont dotés d'une bonne solubilité dans les solvants physiologiquement acceptables usuels, notamment les solutés injectables. Ils peuvent donc être administrés aussi bien par voie orale que par voie injectable, notamment par voie intraveineuse.

Selon l'un de ses aspects, l'invention a donc pour objet les composés de formule (I) : 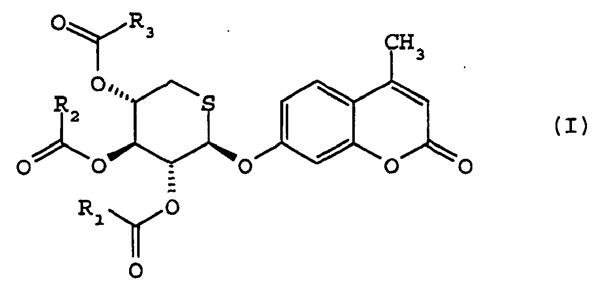 dans laquelle :
R₁, R₂ et R₃ identiques ou différents représentent, chacun indépendamment :
   - un groupe (C₁-C₆)alkyle ,
   - un groupe pyridinyle, ou
   - un groupe -CH₂-NR₄R₅ dans lequel R₄ et R₅ représentent chacun indépendamment un atome d'hydrogène, un groupe (C₁-C₄)alkyle ou bien R₄ et R₅ forment avec l'atome d'azote auquel ils sont liés, un groupe pyrrolidinyle, pipéridinyle, hexahydroazépinyle, morpholinyle ou pipérazinyle,
   à la condition que l'un au moins des substituants R₁, R₂ ou R₃ soit différent du groupe (C₁-C₆)alkyle,
   ainsi que leurs sels, solvates et hydrates, notamment pharmaceutiquement acceptables.

Dans la présente description, on entend par groupe (C₁-C₄) alkyle une chaîne hydrocarbonée saturée ayant de 1 à 4 atomes de carbone, linéaire ou ramifiée. Des exemples de groupes (C₁-C₄) alkyle comprennent les groupes méthyle, éthyle, propyle, butyle, 1-méthyléthyle, 1-méthylpropyle, 2-méthylpropyle ou 1,1-diméthyléthyle. On entend par groupe (C₁-C₆) alkyle, une chaîne hydrocarbonée saturée ayant de 1 à 6 atomes de carbone, linéaire ou ramifiée. Des exemples de groupes (C₁-C₆) alkyle comprennent les groupes précédemment cités ainsi que les groupes pentyle et hexyle.

Par sels , on entend les sels d'addition obtenus par réaction d'un composé de formule I contenant au moins une fonction basique sous sa forme non salifiée, avec un acide minéral ou organique. De préférence, il s'agira de sels d'addition pharmaceutiquement acceptables.

Parmi les acides minéraux convenant pour salifier un composé basique de formule I, on préfère les acides chlorhydrique, bromhydrique, phosphorique et sulfurique. Parmi les acides organiques convenant pour salifier un composé basique de formule I, on préfère les acides méthanesulfonique, et trifluoroacétique.

Parmi les composés de l'invention, on préfère les composés de formule (I)
dans laquelle :
R₁, R₂ et R₃ identiques ou différents représentent, chacun indépendamment :
   - un groupe (C₁-C₄)alkyle,
   - un groupe pyridin-3-yle, ou
   - un groupe -CH₂-NR₄R₅ dans lequel R₄ et R₅ représentent chacun indépendamment un atome d'hydrogène, un groupe (C₁-C₄)alkyle ou bien R₄ et R₅ forment avec l'atome d'azote auquel ils sont liés, un groupe pyrrolidinyle, pipéridinyle ou morpholinyle,
à la condition que l'un au moins des substituants R₁, R₂ ou R₃ soit différent du groupe (C₁-C₄)alkyle,
ainsi que leurs sels, solvates et hydrates, pharmaceutiquement acceptables.

Les composés de formule (I) dans laquelle deux au moins des substituants R₁, R₂ ou R₃ sont identiques, ainsi que leurs sels, solvates et hydrates pharmaceutiquement acceptables, sont également des composés préférés de l'invention.

Les composés de formule (I) dans laquelle R₁, R₂ et R₃ sont identiques, ainsi que leurs sels, solvates et hydrates pharmaceutiquement acceptables, sont aussi des composés préférés de l'invention.

Selon un autre aspect, l'invention concerne un procédé de préparation des composés de formule (I), qui comprend les étapes qui consistent à :
1) faire réagir un dérivé de 5-thio-β-D-xylopyranoside de formule (II) : 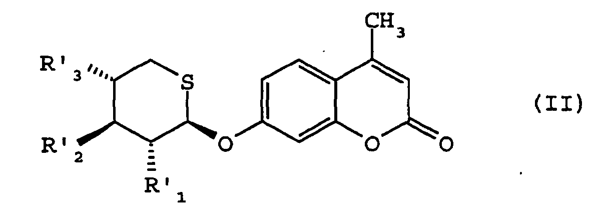 dans laquelle :
   - soit R'₁, R'₂ et R'₃ sont identiques et représentent un groupe hydroxy,
   - soit deux des substituants R'₁, R'₂ ou R'₃ sont identiques et représentent un groupe hydroxy, l'autre substituant R'₁, R'₂ ou R'₃ représente respectivement -OC(O)R₁, -OC(O)R₂ ou -OC(O)R₃ , avec R₁, R₂ et R₃ tels que définis pour (I),
   - soit un des substituants R'₁, R'₂ ou R'₃ représente un groupe hydroxy, les deux autres substituants R'₁, R'₂ ou R'₃ représentent respectivement
   - OC(O)R₁, -OC(O)R₂ ou -OC(O)R₃ , avec R₁, R₂ et R₃ tels que définis pour (I),
   avec un acide approprié de formule (III) : 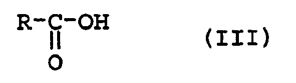 ou un halogènure d'acide approprié de formule (IIIa) : 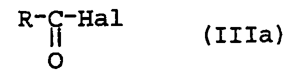 dans laquelle Hal représente un atome d'halogène, notamment le chlore, le fluor et le brome, de préférence le chlore et R représente R₁, R₂ ou R₃ tel que défini pour (I).
2) éventuellement à transformer le composé de formule (I) ainsi obtenu en l'un de ses sels.

La première étape du procédé de l'invention est une étape d'estérification qui peut être réalisée selon les modes opératoires bien connus de l'homme du métier.

Avantageusement, on réalise cette étape d'estérification à l'aide d'un agent de couplage, tel qu'un carbodiimide, par exemple le 1-[3-(dirnéthylamino)propyl]-3-éthylcarbodiimide (EDCI), de préférence en présence de 1- hydroxybenzotriazole (HOBT).

La réaction d'estérification est généralement conduite dans un solvant anhydre, tel que par exemple le diméthylformamide (DMF), la pyridine, le dichlorométhane, en présence d'une base aprotique, telle que la triéthylamine, la 4-diméthylaminopyridine (DMAP) ou leurs mélanges.

De préférence, on opère sous atmosphère inerte et sèche, par exemple sous atmosphère d'azote ou d'argon et en présence d'un capteur d'humidité, tel qu'un tamis moléculaire.

La réaction est avantageusement réalisée entre la température ambiante et la température de reflux du solvant.

La seconde étape du procédé de l'invention est une étape de salification, qui est réalisée selon des méthodes bien connues de l'homme du métier par réaction des composés de formule (I) avec des acides minéraux ou organiques, notamment pharmaceutiquement acceptables, tel que par exemple l'acide chlorhydrique, l'acide méthanesulfonique ou l'acide trifluoroacétique.

Les acides (III) et halogénures d'acide (IIIa) sont des composés commerciaux ou sont préparés selon des méthodes classiques.

Le composé de formule (II) dans lequel R'₁=R'₂=R'₃=OH , dénommé ci-après composé II.1, est décrit dans le brevet EP 421 829. Les autres composés de formule (II) peuvent être obtenus à partir du composé IL .1 par réaction avec un acide ou un halogénure d'acide de formule (III) ou (IIIa), dans lesquelles les groupes R₁, R₂ et R₃ sont éventuellement protégés comme indiqué ci-après.

Les composés de formule (II) peuvent également être obtenus à partir du dérivé de type stannylène de formule (IV) : 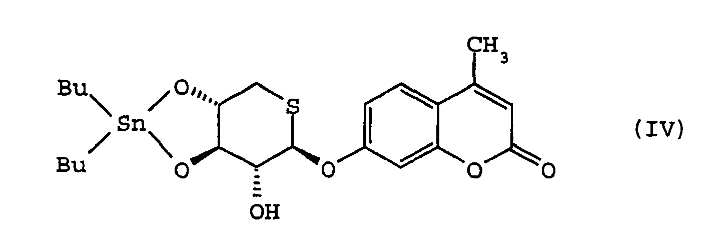 dans laquelle Bu désigne le butyle, selon le mode opératoire décrit par exemple dans *J. Org. Chem.* 1991, 56, 7015.

Les fonctions de type esters du composé (II) peuvent être obtenues sélectivement par action d'un ou deux équivalents d'agents acylants appropriés de formule (IIIa) sur le composé de formule (IV).

Lorsque les radicaux R₁, R₂ et/ou R₃ comportent des fonctions amine libres, il est préférable de les protéger par des groupes protecteurs afin de pouvoir obtenir les composés de formule (I) désirés.

Les réactions de protection et déprotection sont effectuées selon les techniques bien connues de l'homme du métier. Avantageusement, on utilisera comme groupe protecteur de la fonction amine, le groupe *tert*-butoxycarbonyle clivable en milieu acide.

Dans la suite de la description, les composés de formule (II) ainsi protégés seront dénommés composés (I'). Ils sont obtenus par réaction des composés de formule (II) avec un acide ou un halogénure d'acide de formules (III) ou (IIIa) dans lesquelles les groupes R₁, R₂ et R₃ sont protégés.

Les composés selon l'invention ont fait l'objet d'études pharmacologiques.

L'activité antithrombotique des composés selon l'invention a été étudiée *in vivo* chez le rat grâce à un test reproduisant une thrombose veineuse.

L'activité par voie orale a été étudiée selon le protocole décrit dans *Thromb. Haemost*. 1992, 67(1), 176-179. L'activité par voie intraveineuse ou par voie orale a été étudiée selon le protocole opératoire suivant :

L'expérimentation est réalisée sur des rats mâles Wistar, non à jeun, pesant 250 à 280 g et répartis en groupes de 10 animaux chacun. Les produits à tester sont administrés soit par voie orale (tubage) en solution ou en suspension dans du sérum physiologique, soit par injection intraveineuse, en solution dans du sérum physiologique. La concentration des composés est calculée de façon à faire absorber une quantité de solution de 2 ml/kg par voie orale et 1 ml/kg par injection intraveineuse. Une thrombose est induite à un temps T (2, 4 ou 8 heures) après l'administration du produit et le thrombus formé est prélevé et pesé. Pour induire cette thrombose, on réalise une stase veineuse sous hypercoagulation, selon la technique décrite par WESSLER *(J. Applied Physiol.* 1959, 943-946) en utilisant en tant qu'agent hypercoagulant une solution de facteur X activé (Xa), fournie par la société Biogenic, et dosée à 7,5 nKat/kg. L'activité des composés testés a été contrôlée à différentes doses, après qu'ils aient été administrés soit par voie orale (*p*.*o*.) soit par voie intraveineuse (*i*.*v*.). L'induction de la thrombose a été faite 4 heures ou 8 heures après l'administration du composé par voie orale et 2 heures après administration du composé par voie intraveineuse. Pour des doses variant entre 8 et 17 mg, administrées par voie *p.o.,* on a obtenu après 4 heures des pourcentages d'inhibition calculés par rapport au poids d'un thrombus obtenu en l'absence de principe actif dans le sérum physiologique, variant entre 40 et 99 %. Pour des doses variant entre 5 et 7 mg, administrées par voie *i*.*v*., on a obtenu, après 2 heures, des pourcentages d'inhibition, variant entre 40 et 95 %.

A titre d'exemple, le composé selon l'exemple 2A, administré par voie orale à la dose de 13 mg/kg entraîne, après 4 heures, une inhibition de 91% de l'augmentation du poids du thrombus veineux.

Ce même composé, administré par voie intraveineuse à la dose de 5,4 mg/kg entraîne, après 2 heures, une inhibition de 75% de l'augmentation du poids du thrombus.

Ces résultats montrent que les composés selon l'invention présentent une activité antithrombotique, en particulier sur la thrombose veineuse, à la fois par voie orale et par voie intraveineuse.

A titre de comparaison, les composés décrits dans EP 421 829 sont insolubles dans le sérum physiologique et ne peuvent donc pas être administrés par voie intraveineuse.

La présente invention a donc pour objet les composés de formule (I) selon l'invention ainsi que leurs sels, solvates et hydrates pharmaceutiquement acceptables pour leur utilisation en tant que médicament. Les composés de formule (I) ou un de leurs sels, solvates ou hydrates pharmaceutiquement acceptables pourront être utilisés pour la préparation d'un médicament antithrombotique destiné, en particulier, au traitement ou à la prévention des troubles de la circulation veineuse et notamment, pour corriger certains paramètres hématologiques sensibles au niveau veineux.

La présente invention a donc également pour objet des compositions pharmaceutiques contenant un composé de formule (I) ou un de ses sels, solvates ou hydrates pharmaceutiquement acceptables. Ces compositions pharmaceutiques contiennent en général des excipients convenables. Lesdits excipients sont choisis selon la forme pharmaceutique et le mode d'administration souhaités, en particulier orale ou injectable.

Ces compositions pharmaceutiques sont préparées selon les méthodes classiques bien connues de l'homme du métier. Par exemple, les composés selon l'invention peuvent être formulés avec des excipients physiologiquement acceptables pour obtenir une forme injectable à utiliser directement, une forme injectable à préparer extemporanément ou une forme solide pour administration par voie orale telle que, par exemple, une gélule ou un comprimé.

A titre d'exemple, une forme injectable peut être préparée de préférence par lyophilisation d'une solution filtrée et stérilisée contenant le composé selon l'invention et un excipient soluble en quantité nécessaire et suffisante pour obtenir une solution isotonique après addition extemporanée d'eau pour injection. Une forme administrable par voie orale sera de préférence présentée sous forme d'une gélule contenant le composé de l'invention broyé finement ou mieux, micronisé, et mélangé avec des excipients connus de l'homme du métier, tel que par exemple du lactose, de l'amidon prégélatinisé, du stéarate de magnésium.

Afin d'obtenir l'effet thérapeutique ou prophylactique désiré, chaque dose unitaire peut contenir 25 à 500 mg d'au moins un composé selon l'invention.

Dans les exemples qui suivent, on désigne par "préparation" les exemples décrivant la synthèse de composés intermédiaires et par "exemple" ceux décrivant la synthèse de composés de formule (I) selon l'invention. Ces exemples ont pour but d'illustrer l'invention, et ne sauraient en aucun cas en limiter la portée. Les points de fusion sont mesurés au banc Koffler et les valeurs spectrales de Résonance Magnétique Nucléaire sont caractérisées par le déplacement chimique calculé par rapport au TMS, par le nombre de protons associés au signal et par la forme du signal (s pour singlet, d pour doublet, t pour triplet, q pour quadruplet, m pour multiplet). La fréquence de travail et le solvant utilisé sont indiqués pour chaque composé.
Dans ces exemples, l'abréviation DMSO désigne le diméthylsulfoxyde.

### PREPARATION 1

### Acide N-morpholine acétique, composé III.1

### a) N-morpholinoacétate de benzyle

A une solution de 5,03 g de 2-bromoacétate de benzyle dans 75 ml de tétrahydrofurane sous courant d'argon et à température ambiante, 3 ml de morpholine sont ajoutés. Un précipité blanc se forme instantanément. Le mélange est ensuite agité pendant 17 heures. Le sel précipité blanc est filtré et lavé avec 50 ml d'acétate d'éthyle. Le filtrat est lavé avec successivement 50 ml de solution aqueuse saturée en carbonate de sodium, 50 ml d'eau et 50 ml de solution aqueuse saturée en chlorure de sodium. La phase organique est séchée sur sulfate de magnésium, filtrée et évaporée et le liquide jaune résiduel est purifié par chromatographie sur gel de silice (éluant : acétate d'éthyle/hexane, 6/4, v/v). (Rendement = 64 %).

### b) Acide N-morpholine acétique

A une solution de 3,25 g de *N*-morpholinoacétate de benzyle dans 100 ml d'éthanol, 325 mg de palladium sur charbon activé sont ajoutés. Le mélange est ensuite agité sous atmosphère d'hydrogène à température ambiante pendant 17 heures. Le catalyseur est filtré et rincé avec deux fois 50 ml de méthanol. Le filtrat est évaporé pour donner le produit attendu sous forme d'un solide blanc (Rendement = 97 %);
F = 151-155 °C
De la même façon, les composés suivants sont synthétisés :
- acide *N*-pipéridine acétique, composé III.2 ; F = 209-212 °C
- acide *N*-pyrrolidine acétique, composé III.3 ; F = 138-141 °C
- *N,N*-diéthylglycine, composé III.4 ; F = 131-133 °C

### PREPARATION 2

### 4-Méthyl-2-oxo-2H-1-benzopyran-7-yl 3,4-di-O-acétyl-5-thio-β-D-xylopyranoside composé II.2

### 4-Méthyl-2-oxo-2H-1-benzopyran-7-yl 2,4-di-O-acétyl-5-thio-β-D-xylopyranoside composé II.3

a) **4-Méthyl-2-oxo-2*H*-1-benzopyran-7-yl 3,4-di-O-(dibutylstannylène)-5-thio-β-D-xylopyranoside, composé IV**
   Une suspension de 4 g de 4-méthyl-2-oxo-2*H*-1-benzopyran-7-yl 5-thio-β-D-xylopyranoside (composé II.1) et de 6,14 g d'oxyde de dibutylétain dans 200 ml de méthanol est agitée à reflux pendant 4 heures dans un ballon équipé d'un soxhlet muni d'une cartouche contenant du tamis moléculaire 4 Å. Après filtration du mélange réactionnel, le solvant est évaporé sous pression réduite. Le composé IV ainsi obtenu est utilisé tel quel dans les étapes suivantes.
b) A une suspension de 6,25 g de composé IV dans 200 ml de dioxane, une solution de 1,9 g de chlorure d'acétyle dans 20 ml de dioxane est additionnée goutte à goutte. Après 2 heures à 30 °C, le mélange réactionnel est filtré et les solvants sont évaporés sous pression réduite. Une chromatographie sur gel de silice (cyclohexane/acétate d'éthyle, 45/55, v/v) permet de séparer 1,5 g de composé II.2 (34 %) et 660 mg de composé II.3 (14 %).
Composé II.2 : F = 169 °C ; [α]_{D}²² = -162° (c = 0,2, CHCl₃)
Composé II.3 : RMN ¹H (300 MHz, DMSO) : 7,70 (d, 1H) ; 7,17 (d, 1H) ; 7,00 (dd, 1H) ; 6,28 (s, 1H) ; 5,75 (d, 1H, J₁₋₂ = 9 Hz) ; 5,16 (t, 1H) ; 4,77 (m, 1H) ; 3,62 (m, 1H) ; 2,84 (m, 2H) ; 2,40 (s, 3H) ; 2,00 (s, 3H) ; 1,96 (s, 3H).

### PREPARATION 3

### 4-Méthyl-2-oxo-2H-1-benzopyran-7-yl 4-O-(2,2-diméthylpropanoyl)-5-thio-β-D-xylopyranoside, composé II.4

A une suspension de 41 g de composé IV obtenu à la PREPARATION 2 dans 800 ml de dioxane, 19,575 g de chlorure de triméthylacétyle sont additionnés goutte à goutte. Après 2 heures à 30 °C, le mélange réactionnel est filtré. Les filtrats sont repris dans l'acétate d'éthyle, lavés à l'eau, puis séchés sur sulfate de magnésium. Les solvants sont évaporés sous pression réduite et le résidu obtenu est purifié par chromatographie sur une colonne de gel de silice (éluant : cyclohexane/acétate d'éthyle, 55/45, v/v) (Rendement : 59 %); F = 160 °C ; [α]_{D}²³ = - 97° (c = 0,5, CH₃OH).

### PREPARATION 4

### 4-Méthyl-2-oxo-2H-1-benzopyran-7-yl 4-O-acétyl-5-thio-β-D-xylopyranoside, composé II.5

A une solution de 4,4 g de composé IV obtenu à la PREPARATION 2 dans 20 ml de dichlorométhane, on additionne sous atmosphère inerte, 0,57 ml de chlorure d'acétyle, puis après 5 heures d'agitation 0,2 ml supplémentaire. Le mélange réactionnel est agité pendant 3 jours. Après filtration, le composé II.5 est obtenu (Rendement : 61 %) ; F = 210 °C ; [α]²²_{D}= - 68° (c = 0,48 , DMSO).

### EXEMPLE 1

### 4-Méthyl-2-oxo-2H-1-benzopyran-7-yl 2,3,4-tri-O-[(diméthylamino)acétyl]-5-thio-β-D-xylopyranoside

A une solution de 1 g de 4-méthyl-2-oxo-2*H*-1-benzopyran-7-yl 5-thio-β-D-xylopyranoside (composé II.1) dans 25 ml de diméthylformamide (DMF), sous atmosphère d'argon et en présence de tamis moléculaire 3Å, sont additionnés successivement 2 ml de triéthylamine, 2,84 g de chlorhydrate de 1-[3-(diméthylamino)propyl]-3-éthylcarbodiimide (EDCI), 0,56 g de 4-diméthylaminopyridine (DMAP), 30 ml d'une solution 0,5 M d'hydrate de 1-hydroxybenzotriazole (HOBT) dans le DMF et 1,53 g de *N,N*-diméthylglycine. Le mélange réactionnel est ensuite agité pendant 18 heures. Le précipité est filtré et rincé avec 30 ml d'acétate d'éthyle. Le filtrat est concentré et l'huile orange résiduelle est reprise dans 100 ml d'eau et 15 ml de triéthylamine. La phase aqueuse est extraite avec de l'acétate d'éthyle (100 ml + 50 ml). Les phases organiques sont réunies et lavées avec 50 ml d'eau puis 50 ml de solution aqueuse saturée en chlorure de sodium. La phase organique est ensuite séchée sur sulfate de magnésium, filtrée et concentrée. Le solide jaune résiduel est chromatographié sur gel de silice (éluant : acétate d'éthyle/triéthylamine, 99/1, v/v puis dichlorométhane/méthanol/triéthylamine 9/1/0,1 v/v/v). La fraction collectée est évaporée et la mousse solide jaunâtre résiduelle est reprise dans de l'éther diéthylique. Le produit désiré est filtré et rincé avec de l'éther diéthylique. (Rendement = 72 %) ; F = 112-114 °C.
Le trichlorhydrate correspondant (EXEMPLE 1A) est préparé en additionnant 7,5 ml d'une solution 1M de chlorure d'hydrogène dans de l'éther diéthylique, à une solution de 0,7 g du composé de l'EXEMPLE 1 dans 3 ml de méthanol anhydre à 0°C. Une pâte jaunâtre se forme alors instantanément. Le mélange est ensuite dilué avec 5 ml d'éther diéthylique anhydre, agité à 0°C pendant 1 heure puis concentré pour obtenir un solide jaunâtre ;
F = 196 °C ; [α]²⁹_{D}= - 32° (c = 0,55 , CH₃OH).

En procédant selon le même mode opératoire, à partir de l'acide (III) correspondant, et en présence ou non de HOBT, les EXEMPLES 2 à 5A présentés dans le TABLEAU 1 ci-après, sont préparés.

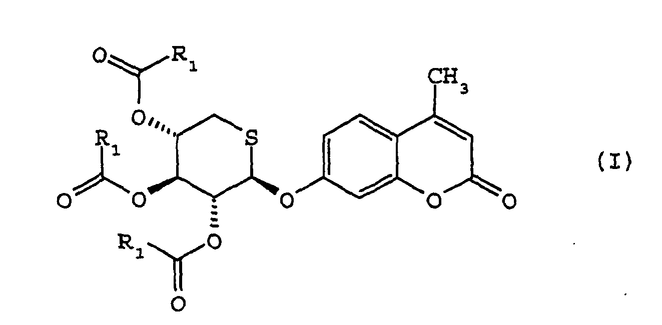

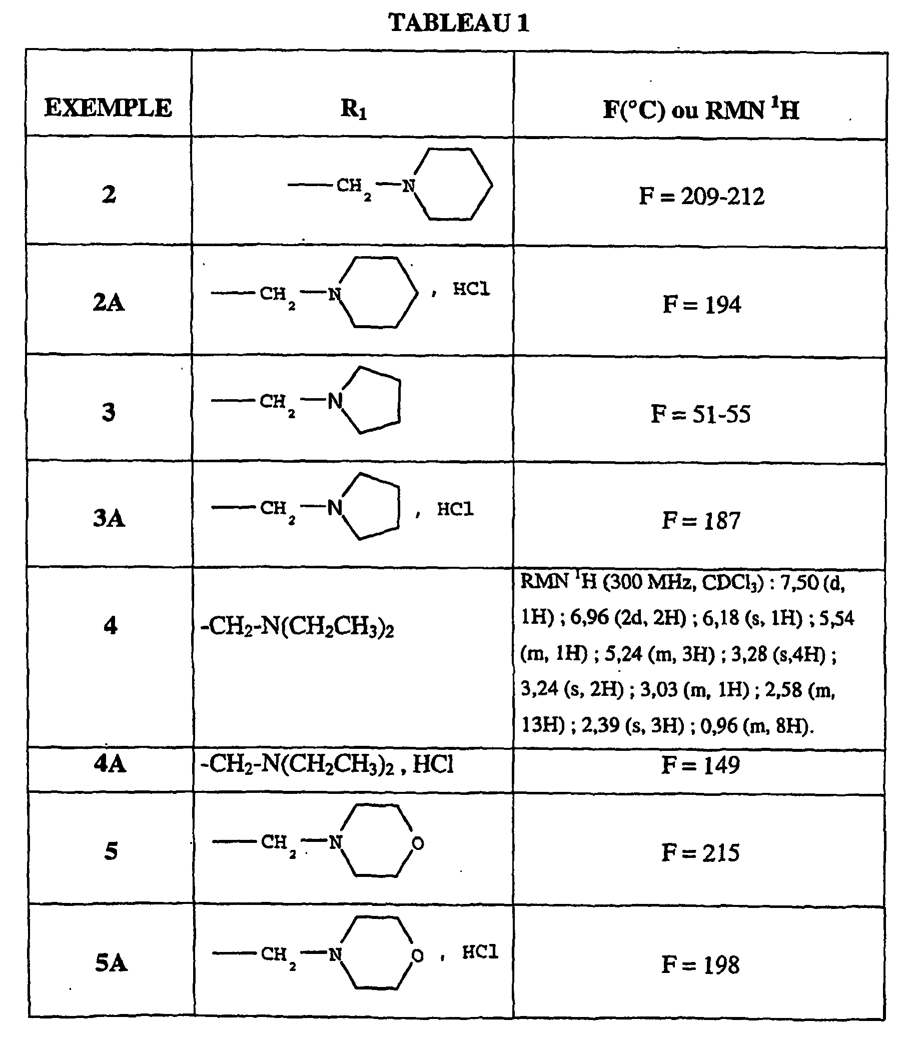

### EXEMPLE 6

### 4-Méthyl-2-oxo-2H-1-benzopyran-7-yl 2,3,4-tri-O-(pyridin-3-ylcarbonyl)-5-thio-β-D-xylopyranoside

4 g de composé II.1 et 11 g de chlorure de nicotinoyle chlorhydrate dans 50 ml de pyridine sont agités à température ambiante pendant 48 heures. Le mélange réactionnel est filtré et les solvants sont évaporés. Le résidu obtenu est lavé à l'eau puis purifié par chromatographie sur gel de silice en éluant avec un mélange dichlorométhane/méthanol/ammoniaque, 95/5/0,5, (v/v/v). La poudre blanche obtenue est séchée sous pression réduite.
[α]_{D}²⁶= 37° (c = 0,6, CHCl₃)

### EXEMPLE 7

### 4-Méthyl-2-oxo-2H-1-benzopyran-7-yl 3-O-[(diméthylamino)acétyl]-2,4-di-O-acétyl-5-thio-β-D-xylopyranoside

Une solution de 620 mg de composé II.3 dans 20 ml de dichlorométhane stabilisé à l'amylène en présence de 563 mg de EDCI, de 302 mg de *N,N-*diméthylglycine et de 89 mg de DMAP est chauffée à reflux pendant 1 heure. Le mélange réactionnel est alors filtré. Le filtrat est lavé avec une solution de chlorure d'ammonium et de chlorure de sodium puis la phase organique est séchée sur sulfate de magnésium. Les solvants sont évaporés sous pression réduite et le résidu obtenu est purifié par chromatographie sur gel de silice (éluant: toluène/isopropanol, 10/1, v/v) (Rendement = 76 %) ;
F = 214 °C
RMN ¹H (300 MHz, DMSO) : 6,00 (d, 1H, J₁₋₂ = 8,4 Hz), 5,32 (2t, 2H) ; 4,98 (m, 1H); 3,15 (m, 3H) ; 2,94 (m, 1H) ; 2,40 (s, 3H) ; 2,21 (s, 6H) ; 2,00 et 1,92 (2s, 6H).

Le méthanesulfonate correspondant (EXEMPLE 7A) est obtenu comme suit : 466 mg du composé de l'EXEMPLE 7 sont mis en solution dans 30 ml d'acétate d'éthyle. A cette solution, une solution de 92 mg d'acide méthanesulfonique dans 4 ml de tétrahydrofurane est additionnée. Après une heure, le mélange réactionnel est filtré (Rendement = 79 %) ; F = 203 °C ; [α]_{D}^{21,5}= - 56° (c = 0,44 ,CHCl₃)

### EXEMPLE 8

### 4-Méthyl-2-oxo-2H-1-benzopyran-7-yl 2-O-[(diméthylamino)acétyl]-3,4-di-O-acétyl-5-thio-β-D-xylopyranoside

Le composé de l'EXEMPLE 8 est préparé à partir du composé II.2 selon un mode opératoire analogue à celui de l'EXEMPLE 7. (Rendement = 92 %) ;
F = 102 °C.
RMN ¹H (300 MHz, DMSO) : 6,02 (d, 1H, J₁₋₂ = 8,9 Hz) ; 5,4 (t, 1H) ; 5,26 (t, 1H) ; 5,00 (m, 1H) ; 3,08 (m, 3H) ; 2,95 (m, 1H) ; 2,40 (s, 3H) ; 2,21 (s, 6H) ; 2,01 et 1,99 (2s, 6H).

### EXEMPLE 8A

### 4-Méthyl-2-oxo-2H-1-benzopyran-7-yl 2-O-[(diméthylamino)acétyl]-3,4-di-O-acétyl-5-thio-β-D-xylopyranoside, méthanesulfonate

Le composé de l'EXEMPLE 8A est préparé selon le même mode opératoire que l'EXEMPLE 7A (Rendement = 76 %) ; F = 110 °C ;
[α]_{D}²³= - 44° (c = 0,29 , CHCl₃).

### EXEMPLE 9

### 4-Méthyl-2-oxo-2H-1-benzopyran-7-yl 2,3-bis-O-[(diméthylamino)acétyl]-4-O-(2,2-diméthylpropanoyl)-5-thio-β-D-xylopyranoside, diméthanesulfonate

2 g de composé II.4 sont mis en solution dans 50 ml de dichlorométhane en présence de tamis moléculaire 4 Å. 3 g de EDCI, 0,6 g de DMAP et 1,63 g de *N,N*-diméthylglycine sont additionnés. Après 4 heures sous agitation, le mélange réactionnel est filtré. Le filtrat est lavé avec une solution de chlorure d'ammonium et à l'eau, puis est séché sur sulfate de magnésium. Les solvants sont évaporés sous pression réduite. Le résidu obtenu est purifié par chromatographie sur gel de silice (éluant : dichlorométhane/méthanol, 10/1, v/v). 2,45 g du composé obtenu sont dissous dans 50 ml d'acétate d'éthyle anhydre puis 600 µl d'acide méthanesulfonique en solution dans 13 ml de THF sont ajoutés goutte à goutte. Après 45 minutes sous agitation, le précipité est filtré et séché.
Rendement = 69 % ; F = 236 °C ;
[α]_{D}²⁶= - 19° (c = 0,41 , DMSO).

### EXEMPLE 10

### 4-Méthyl-2-oxo-2H-1-benzopyran-7-yl 4-O-acétyl-2,3-bis-O-[(diméthylamino)acétyl]-5-thio-β-D-xylopyranoside, diméthanesulfonate

Une solution de 500 mg du composé II.5 dans 20 ml de dichlorométhane est agitée sous atmosphère inerte en présence de 838 mg de EDCI de 167 mg de DMAP et de 450 mg de *N,N*-diméthylglycine pendant 3 heures. Le mélange réactionnel est alors lavé à l'eau et la phase organique est concentrée sous pression réduite. Le résidu est purifié par chromatographie sur gel de silice en éluant avec un mélange dichlorométhane/méthanol, 10/1, (v/v). 490 mg du composé obtenu sont repris dans 10 ml d'acétate d'éthyle et 135 µl d'acide méthanesulfonique dans 2 ml de tétrahydrofurane sont additionnés. Le précipité est filtré et séché sous pression réduite (rendement = 50 %) ; F = 130 °C ; [α]_{D}²²= - 14°(c = 0,39, DMSO)

### EXEMPLE 11

### 4-Méthyl-2-oxo-2H-1-benzopyran-7-yl 2,3,4-tri-O-(aminoacétyl)-5-thio-β-D-xylopyranoside, tris(trifluoroacétate)

**a) 4-méthyl-2-oxo-2*H*-1-benzopyran-7-yl 2,3,4-tri-O-[(*N*-*tert-*butoxycarbonylamino)acétyl]-5-thio-β-D-xylopyranoside, composé I'.1**
   Une suspension de 325 mg de composé II.1 dans 15 ml de diméthylformamide est chauffée jusqu'à dissolution, puis ramenée à température ambiante. On y ajoute sous agitation 578 mg de *N-tert*-butoxycarbonylglycine, 690 mg de chlorhydrate de 1-(3-diméthylaminopropyl)-3-éthylcarbodiimide et 184 mg de diméthylaminopyridine. Le mélange réactionnel est maintenu dans ces conditions pendant 20 heures et le diméthylformamide est alors évaporé sous pression réduite. Le résidu est repris dans du dichlorométhane. Cette phase organique est lavée successivement avec une solution aqueuse d'acide chlorhydrique 1N, une solution d'hydrogénocarbonate de sodium, une solution saturée en chlorure de sodium puis est séchée sur sulfate de magnésium. Les solvants de la phase organique sont évaporés sous pression réduite puis le résidu est purifié par chromatographie sur gel de silice (toluène/acétate d'éthyle, 7/3, v/v) (Rendement = 80 %).
   RMN ¹H (300 MHz, DMSO) : 7,73 (d, 1H) ; 7,16 (m, 4H) ; 7,00 (dd, 1H) ; 6,29 (s, 1H) ; 6,00 (d, 1H, J=8,8 Hz) ; 5,40 (t, 1H) ; 5,30 (t, 1H) ; 5,07 (m, 1H) ; 3,68 (dd, 2H) ; 3,11 (t, 1H) ; 2,94 (dd, 1H) ; 1,38 (s, 9H).
b) 630 mg du composé I'.1 sont dissous dans 75 ml d'acide trifluoroacétique (TFA) et 25 ml de dichlorométhane stabilisé à l'amylène. Le mélange est agité à température ambiante pendant 1 heure puis les solvants sont évaporés sous pression réduite. Le résidu est précipité dans l'éther diéthylique et filtré. Ce précipité est alors purifié par chromatographie sur gel de silice (eau/acétonitrile/TFA, 4/1/0,001, v/v/v). La fraction contenant l'amine déprotégée est lyophilisée 2 fois (Rendement = 32 %) ;
   F = 136 °C ;
   [α]_{D}²⁴= - 18°(c = 0,155, CH₃OH)

### EXEMPLE 11A

### 4-méthyl-2-oxo-2H-1-benzopyran-7-yl 2,3,4-tri-O-(aminoacétyl)-5-thio-β-D-xylopyranoside, trichlorhydrate

A une solution de 883 mg de composé I'.1 dans 5 ml d'éthanol anhydre sous atmosphère d'argon et à température ambiante, sont additionnés 40 ml d'une solution 1M de chlorure d'hydrogène dans de l'éther diéthylique. Après 18 heures d'agitation, le précipité blanc obtenu est filtré puis rincé à l'éther diéthylique anhydre.
F = 180 °C
[α]_{D}²⁶= - 25°(c = 0,42, CH₃OH)

### EXEMPLE 12

### 4-méthyl-2-oxo-2H-1-benzopyran-7-yl 2,3,4-tri-O-[(méthylamino)acétyl]-5-thio-β-D-xylopyranoside, trichlorhydrate

Le composé de l'EXEMPLE 12 est préparé à partir du composé II.1 et de la *N*-(*tert*-butoxycarbonyl)-*N*-méthylglycine, selon un mode opératoire analogue à celui des EXEMPLES 11 et 11A.
F = 195 °C;
[α]_{D}²³= - 26°(c = 0,36, CH₃OH).

## Revendications

1. Composés de formule (I) : 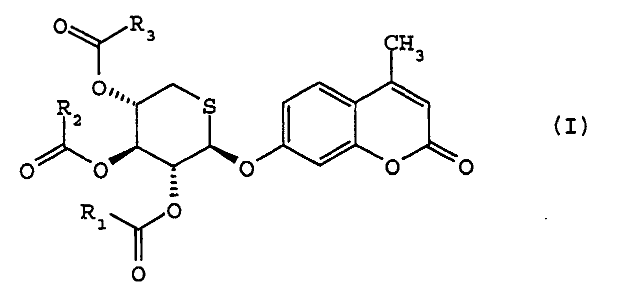 dans laquelle :
R₁, R₂ et R₃ identiques ou différents représentent, chacun indépendamment :
- un groupe (C₁-C₆)alkyle,
- un groupe pyridinyle, ou
- un groupe -CH₂-NR₄R₅ dans lequel R₄ et R₅ représentent chacun indépendamment un atome d'hydrogène, un groupe (C₁-C₄)alkyle ou bien R₄ et R₅ forment avec l'atome d'azote auquel ils sont liés, un groupe pyrrolidinyle, pipéridinyle, hexahydroazépinyle, morpholinyle ou pipérazinyle,
à condition que l'un au moins des substituants R₁, R₂ ou R₃ soit différent du groupe (C₁-C₆)alkyle,
ainsi que leurs sels, solvates et hydrates, notamment pharmaceutiquement acceptables.

2. Composés selon la revendication 1, **caractérisés en ce que** R₁, R₂ et R₃ identiques ou différents représentent, chacun indépendamment :
- un groupe (C₁-C₄)alkyle,
- un groupe pyridin-3-yle, ou
- un groupe -CH₂-NR₄R₅ dans lequel R₄ et R₅ représentent chacun indépendamment un atome d'hydrogène, un groupe (C₁-C₄)alkyle ou bien R₄ et R₅ forment avec l'atome d'azote auquel ils sont liés, un groupe pyrrolidinyle, pipéridinyle ou morpholinyle, à condition que l'un au moins des substituants R₁, R₂ ou R₃ soit différent du groupe (C₁-C₄)alkyle.

3. Composés selon l'une des revendications 1 ou 2, **caractérisés en ce que** deux au moins des substituants R₁, R₂ ou R₃ sont identiques.

4. Composés selon la revendication 3, **caractérisés en ce que** R₁, R₂ et R₃ sont identiques.

5. Composé selon l'une quelconque des revendications 1 à 4 pour son utilisation en tant que médicament.

6. Utilisation d'un composé selon l'une quelconque des revendications 1 à 4 pour la préparation d'un médicament antithrombotique.

7. Utilisation selon la revendication 6 pour la préparation d'un médicament destiné au traitement ou à la prévention des troubles de la circulation veineuse.

8. Compositions pharmaceutiques contenant à titre de principe actif un composé selon l'une quelconque des revendications 1 à 4 en association avec un excipient pharmaceutiquement acceptable.

9. Compositions pharmaceutiques selon la revendication 8, **caractérisées en ce qu'**elles sont sous forme injectable.

10. Procédé pour la préparation d'un composé selon l'une quelconque des revendications 1 à 5, **caractérisé en ce qu'**il comprend les étapes qui consistent à :
1) faire réagir un dérivé de 5-thio-β-D-xylopyranoside de formule (II) : 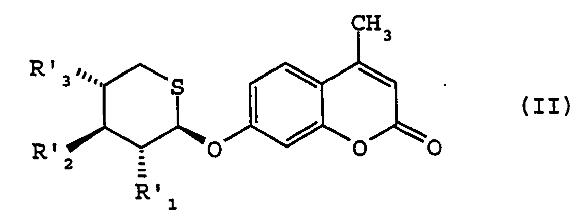 dans laquelle :
- soit R'₁, R'₂ et R'₃ sont identiques et représentent un groupe hydroxy,
- soit deux des substituants R'₁, R'₂ ou R'₃ sont identiques et représentent un groupe hydroxy, l'autre substituant R'₁, R'₂ ou R'₃ représente respectivement -OC(O)R₁, -OC(O)R₂ ou -OC(O)R₃ , avec R₁, R₂ et R₃ tels que définis pour (I),
- soit un des substituants R'₁, R'₂ ou R'₃ représente un groupe hydroxy, les deux autres substituants R'₁, R'₂ ou R'₃ représentent respectivement -OC(O)R₁, -OC(O)R₂ ou -OC(O)R₃ , avec R₁, R₂ et R₃ tels que définis pour (I),
avec un acide approprié de formule (III) : 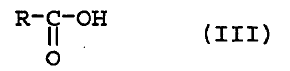
ou un halogènure d'acide approprié de formule (IIIa) : 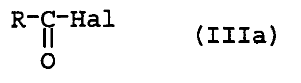 dans laquelle Hal représente un atome de d'halogène, notamment le chlore, le fluor et le brome, de préférence le chlore et R représente R₁, R₂ ou R₃ tel que défini pour (I).
2) éventuellement à transformer le composé de formule (I) ainsi obtenu en l'un de ses sels.

## Claims

1. Compounds of formula (I): 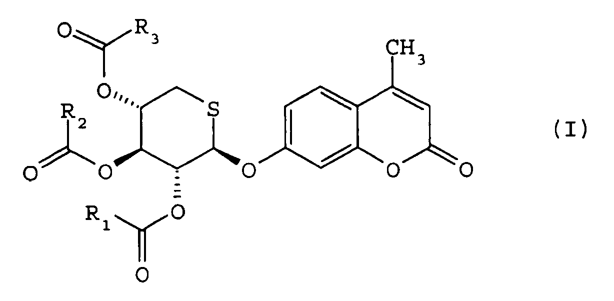 in which:
R₁, R₂ and R₃, which are identical or different, are each independently:
- a (C₁-C₆)alkyl group,
- a pyridinyl group or
- a group -CH₂-NR₄R₅, in which R₄ and R₅ are each independently a hydrogen atom or a (C₁-C₄)alkyl group, or alternatively R₄ and R₅ form, with the nitrogen atom to which they are bonded, a pyrrolidinyl, piperidinyl, hexahydroazepinyl, morpholinyl or piperazinyl group,
with the proviso that at least one of the substituents R₁, R₂ or R₃ is other than a (C₁-C₆)alkyl group,
and their salts, solvates and hydrates, especially those which are pharmaceutically acceptable.

2. Compounds according to claim 1, **characterized in that** R₁, R₂ and R₃, which are identical or different, are each independently:
- a (C₁-C₄)alkyl group,
- a pyridin-3-yl group or
- a group -CH₂-NR₄R₅ in which R₄ and R₅ are each independently a hydrogen atom or a (C₁-C₄)alkyl group, or alternatively R₄ and R₅ form, with the nitrogen atom to which they are bonded, a pyrrolidinyl, piperidinyl or morpholinyl group,
with the proviso that at least one of the substituents R₁, R₂ and R₃ is other than a (C₁-C₄)alkyl group.

3. Compounds according to claim 1 or 2, **characterized in that** at least two of the substituents R₁, R₂ and R₃ are identical.

4. Compounds according to claim 3, **characterized in that** R₁, R₂ and R₃ are identical.

5. Compound according to any one of claims 1 to 4 for use as a drug.

6. Use of a compound according to any one of claims 1 to 4 for the preparation of an antithrombotic drug.

7. Use according to claim 6 for the preparation of a drug intended for the treatment or prevention of disorders of the venous circulation.

8. Pharmaceutical compositions in which a compound according to any one of claims 1 to 4 is present as the active principle, in association with a pharmaceutically acceptable excipient.

9. Pharmaceutical compositions according to claim 8, **characterized in that** they are in injectable form.

10. Method for the preparation of a compound according to any one of claims 1 to 5 **characterized in that** it comprises the steps consisting in:
1) reacting a 5-thio-β-D-xylopyranoside derivative of formula (II): 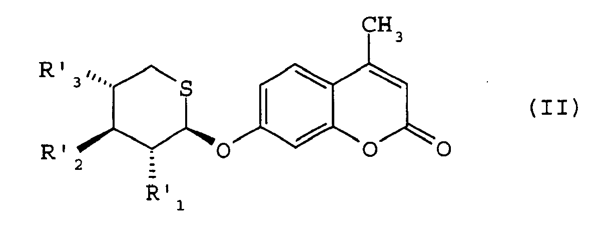 in which:
- either R'₁, R'₂ and R'₃ are identical and are a hydroxyl group,
- or two of the substituents R'₁, R'₂ and R'₃ are identical and are a hydroxyl group and the other substituent R'₁, R'₂ or R'₃ is respectively -OC(O)R₁, -OC(O)R₂ or -OC(O)R₃, where R₁, R₂ and R₃ are as defined for (I),
- or one of the substituents R'₁, R'₂ and R'₃ is a hydroxyl group and the other two substituents R'₁, R'₂ and R'₃ are respectively -OC(O)R₁, -OC(O)R₂ or -OC(O)R₃, where R₁, R₂ and R₃ are as defined for (I),
with an appropriate acid of formula (III): 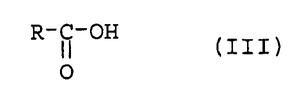 or an appropriate acid halide of formula (IIIa): 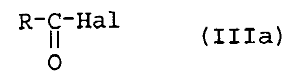 in which Hal is a halogen atom, especially chlorine, fluorine or bromine and preferably chlorine, and R is R₁, R₂ or R₃ as defined for (I); and
2) optionally converting the resulting compound of formula (I) to one of its salts.

## Patentansprüche

1. Verbindungen der Formel (I): 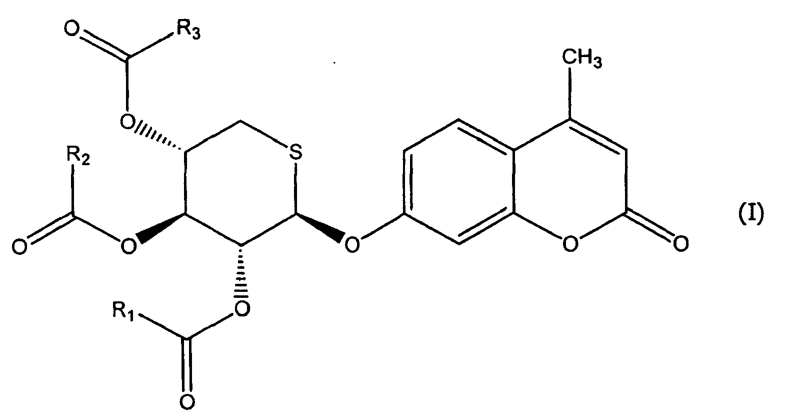 wobei:
R₁, R₂ und R₃ identisch oder verschieden und jeweils unabhängig voneinander
- eine (C₁-C₆)-Alkylgruppe,
- eine Pyridinylgruppe, oder
- eine -CH₂-NR₄R₅-Gruppe darstellen, wobei R₄ und R₅ jeweils unabhängig voneinander ein Wasserstoffatom oder eine (C₁-C₄) Alkylgruppe darstellen bzw. R₄ und R₅ mit dem Stickstoffatom, an das sie gebunden sind, eine Pyrrolidinyl-, eine Piperidinyl-, eine Hexahydroazepinyl-, eine Morpholinyl- oder eine Piperazinyl-Gruppe bilden,
unter der Voraussetzung, dass mindestens einer der Substituenten R₁, R₂ oder R₃ nicht eine (C₁-C₆)-Alkylgruppe ist,
sowie deren Salze, Solvate und Hydrate, insbesondere pharmazeutisch geeignete.

2. Verbindungen gemäß Anspruch 1, **dadurch gekennzeichnet, dass** R₁, R₂ und R₃ identisch oder verschieden und jeweils unabhängig voneinander
- eine (C₁-C₄) Alkylgruppe,
- eine Pyridin-3-ylgruppe, oder
- eine -CH₂-NR₄R₅-Gruppe darstellen, wobei R₄ und R₅ jeweils unabhängig voneinander ein Wasserstoffatom oder eine (C₁-C₄)-Alkylgruppe darstellen bzw. R₄ und R₅ mit dem Stickstoffatom, an das sie gebunden sind, eine Pyrrolidinyl-, eine Piperidinyl- oder eine Morpholinylgruppe bilden, unter der Voraussetzung, dass mindestens einer der Substituenten R₁, R₂ oder R₃ nicht eine (C₁-C₄)-Alkylgruppe ist.

3. Verbindungen gemäß einem der Ansprüche 1 oder 2, **dadurch gekennzeichnet, dass** zumindest zwei der Substituenten R₁, R₂ oder R₃ identisch sind.

4. Verbindungen gemäß Anspruch 3, **dadurch gekennzeichnet, dass** R₁, R₂ und R₃ identisch sind.

5. Verbindung gemäß einem der Ansprüche 1 bis 4 zur Verwendung als Medikament.

6. Verwendung einer Verbindung gemäß einem der Ansprüche 1 bis 4 zur Herstellung eines Antithrombose-Medikaments.

7. Verwendung gemäß Anspruch 6 zur Herstellung eines Medikaments, das zur Behandlung oder zur Prävention von Störungen des Blutkreislaufs in den Venen bestimmt ist.

8. Pharmazeutische Zusammensetzungen, die eine Verbindung gemäß einem der Ansprüche 1 bis 4 als aktiven Wirkstoff in Verbindung mit einem pharmazeutisch geeigneten Vehikel enthalten.

9. Pharmazeutische Verbindungen gemäß Anspruch 8, **dadurch gekennzeichnet, dass** sie in einer injizierbaren Form vorliegen.

10. Verfahren zur Herstellung einer Verbindung gemäß einem der Ansprüche 1 bis 5, **dadurch gekennzeichnet, dass** es folgende Schritte umfasst:
1) Reaktion eines Derivats von 5-Thio-β-D-xylopyranosid der Formel (II): 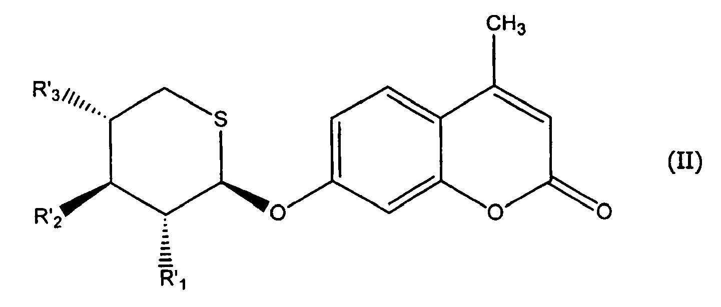 wobei:
- entweder R'₁, R'₂ und R'₃ identisch sind und eine Hydroxylgruppe darstellen,
- oder zwei der Substituenten R'₁, R'₂ oder R'₃ identisch sind und eine Hydroxylgruppe darstellen und der andere Substituent R'₁, R'₂ oder R'₃ jeweils
- OC(O)R₁, -OC(O)R₂ oder -OC(O)R₃ darstellt, mit R₁, R₂ und R₃ wie für (I) definiert,
- oder einer der Substituenten R'₁, R'₂ oder R'₃ eine Hydroxylgruppe darstellt und die beiden anderen Substituenten R'₁, R'₂ oder R'₃ jeweils -OC(O)R₁, -OC(O)R₂ oder -OC(O)R₃ darstellen, mit R₁, R₂ und R₃ wie für (I) definiert, mit einer Säure gemäß Formel (III): 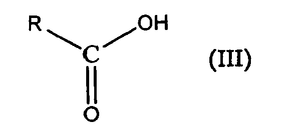
oder einem Säurehalogenid gemäß der Formel (IIIa): 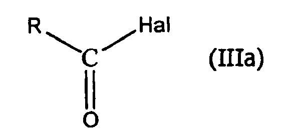 wobei Hal ein Halogenatom darstellt, insbesondere Chlor, Fluor oder Brom, vorzugsweise Chlor, und R R₁, R₂ oder R₃ wie für (I) definiert darstellt.
2) Gegebenenfalls die Umwandlung der Verbindung der Formel (I), die auf diese Art erhalten wurde, in eines ihrer Salze.
